Europäisches Patentamt

European Patent Office

Office européen des brevets

(11) Veröffentlichungsnummer: **0 582 169 A2**

# EUROPÄISCHE PATENTANMELDUNG

(21) Anmeldenummer: 93111920.0

(22) Anmeldetag: 26.07.93

(51) Int. Cl.⁵: **A61K 31/44**, A61K 47/32, A61K 9/08

(30) Priorität: 05.08.92 IT MI921930

(43) Veröffentlichungstag der Anmeldung:
09.02.94 Patentblatt 94/06

(84) Benannte Vertragsstaaten:
AT BE CH DE DK ES FR GB GR IE IT LI LU MC
NL PT SE

(71) Anmelder: **Bayer S.p.A.**
**Viale Certosa 126**
**I-20156 Milano(IT)**

(72) Erfinder: **Ciceri, Silvana**
**Via Galilei 1**
**I-22100 Como(IT)**
Erfinder: **Dondi, Gilberto**
**Via Dei Tigli 13**
**I-20095 Cusano Milanino (Milano)(IT)**
Erfinder: **Scurati, Paolo**
**Via Card. Riboldi 5**
**I-20037 Paderno Dugnano (Milano)(IT)**

(74) Vertreter: **Müller, Gerhard, Dr.**
**BAYER AG**
**Konzernverwaltung RP**
**Patente Konzern**
**D-51368 Leverkusen (DE)**

(54) **Pharmazeutische Zubereitungen zur oralen Verabreichung von Dihydropyridinen in Getränkform.**

(57) Es werden pharmazeutische Zubereitungen zur oralen Verabreichung von Dihydropyridinen in Getränkform beschrieben.

Solche Zubereitungen sind dadurch gekennzeichnet, daß sie ein Copräzipitat aus im wesentlichen amorphem Dihydropyridin mit einem geeigneten pharmakologisch unbedenklichen Polymerisat enthalten. Es werden auch die entsprechenden Herstellungsverfahren beschrieben.

EP 0 582 169 A2

Rank Xerox (UK) Business Services
(3.10/3.09/3.3.4)

Die Verabreichung von pharmazeutischen Zubereitungen auf der Basis von Dihydropyridinen stellt ein ziemlich heikles Problem dar, wegen der äußerst geringen Löslichkeit dieser chemischen Verbindungen.

Das gewöhnlich verwendete Verfahren zur Erhaltung einer genügenden biologischen Verfügbarkeit basiert auf der Verabreichung solcher Dihydropyridinen in amorpher oder mycellarer Form, unter welchen Nimodipin, Nifedipin und Nisoldipin die bekanntesten Vertreter sind.

Der amorphe Zustand wird üblicherweise durch Zubereitungen aus Copräzipitaten mit pharmakologisch unbedenklichen Polymerisaten, wie Polyvinylpyrrolidon (PVP) erhalten, während die Bildung von Mycellen üblicherweise durch Verdünnung von Lösungen, die hohe Gehalte an Tensiden aufweisen und Alkohole als Hilfsmittel enthalten, erhalten wird.

Das gemeinsame Problem ist in beiden Fällen, wie man den Kristallbildungsvorgängen vorbeugen kann, da sie die biologische Verfügbarkeit einiger Dihydropyridinen, worunter Nimodipin, deswegen dramatisch beeinflussen können, da die amorphe und kristalline Form stark unterschiedlich löslich sind.

Unter den verschiedenen verfügbaren pharmazeutischen Formen von Nimodipin, weist das Tropfenpräparat bemerkenswerte und wertvolle Vorteile hinsichtlich der Einnahme dieser Zubereitung, die für die Behandlung einer ungenügenden Hirnzirkulation dient, seitens älteren Patienten, die die Hauptgruppe der in Frage kommenden Patienten darstellt.

Eine schwache Seite des pharmazeutischen Tropfenpräparats besteht aus der Notwendigkeit, daß der Patient die Tropfen selbst dosieren (d.h. zählen) muß. Diese anscheinend einfache Tätigkeit kann in der Tat ein nicht unbeträchtliches Hindernis für einen älteren Patienten sein und als sehr beschwerlich wahrgenommen werden.

Unter den verschiedenen möglichen Lösungen hat sich am geeignetesten diejenige gezeigt, die aus der Zubereitung einer in Wasser dispergierbaren Einzeldosis besteht, die organoleptisch so eingestellt wird, daß der unangenehme Geschmack des Wirkstoffes verdeckt wird. Ein Orangensaft-Granulat und der entsprechende Aromastoff haben sich als sehr zweckmäßig dafür bewiesen. Auch die Anwesenheit eines Brausesystems trägt zur Annehmlichkeit der Zubereitung und zur sicheren Dispersion des Wirkstoffs bei.

Damit die Auflösung des Dihydropyridins, Z. B. des Nimodipins gewährleistet wird, wurde in die Formulierung das Nimodipin/PVP Copräzipitat eingesetzt, wobei die PVP-Menge so bemessen wird, daß sie zur sicheren Erhaltung des amorphen Zustandes des Nimodipins ausreicht, ohne eine unerwünschte Schaumbildung während der Brauseaktivität herbeizuführen.

Das Dihydropyridin/PVP, insbesondere das Nimodipin/PVP-Copräzipitat läßt sich nach verschiedenen Verfahrensweisen, die nachfolgend erläutert werden, herstellen.

- Auflösung von Nimodipin (1 Teil) und PVP (3 Teile) in organischem Lösungsmittel, z.B. Aceton (3 Teile), mit darauffolgender Trocknung im Ofen unter vermindertem Druck.
- Zerstäubung einer Lösung von Nimodipin und PVP in organischem Lösungsmittel.
- Lösung von Nimodipin (1 Teil) und PVP 25 (3 Teile) in Tetrahydrofuran (3 Teile). Ausfällung mittels Petrolether (7 Teile). Filtration und Trocknung des Copräzipitates.
- Trocknung einer Lösung von Nimodipin und PVP in organischem Lösungsmittel in einem Fließbettapparat unter vermindertem Druck.
- Gefriertrocknung einer Lösung von Nimodipin und PVP in einem geeigneten organischen Lösungsmittel, wie tert.-Butylalkohol, usw.

Das optimale Nimodipin/PVP-Verhältnis wurde durch Untersuchung des Lösungsprofils einer 15 mg Nimodipin äquivalenten Copräzipitatmenge, in den aus den verschiedenen Pharmakopöen bekannten, dazu geeigneten Apparaten, bestimmt.

Die Untersuchungsbedingungen sind:

Mediumvolumen 1300 ml 0,1N HCl

T° = 37 °C

Drehzahl des Rührblattes: 50 UpM.

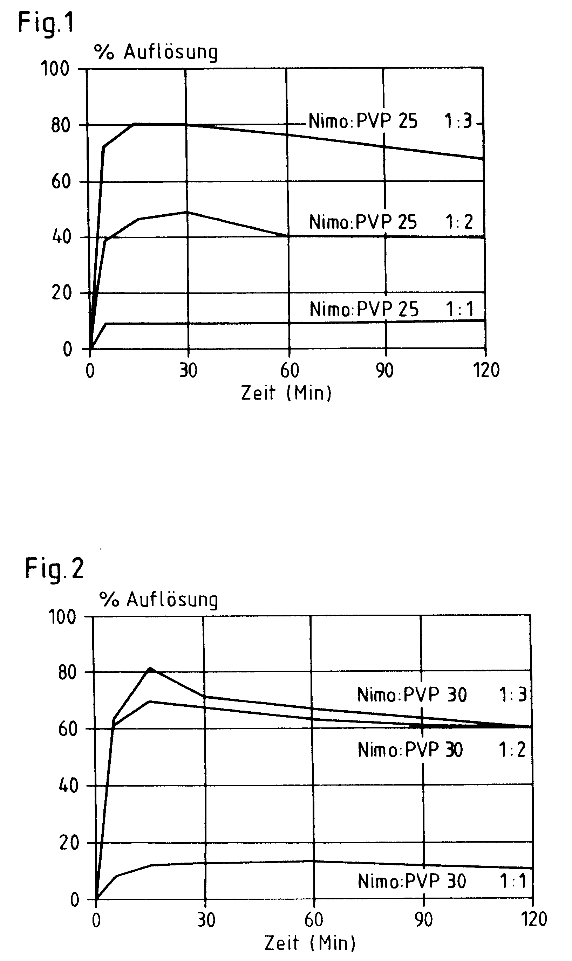

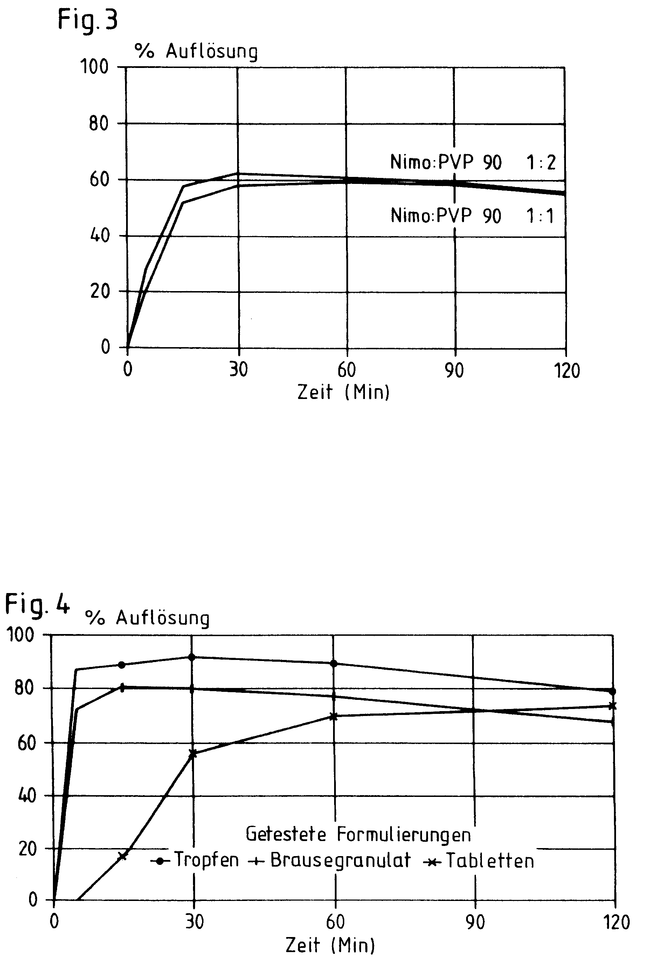

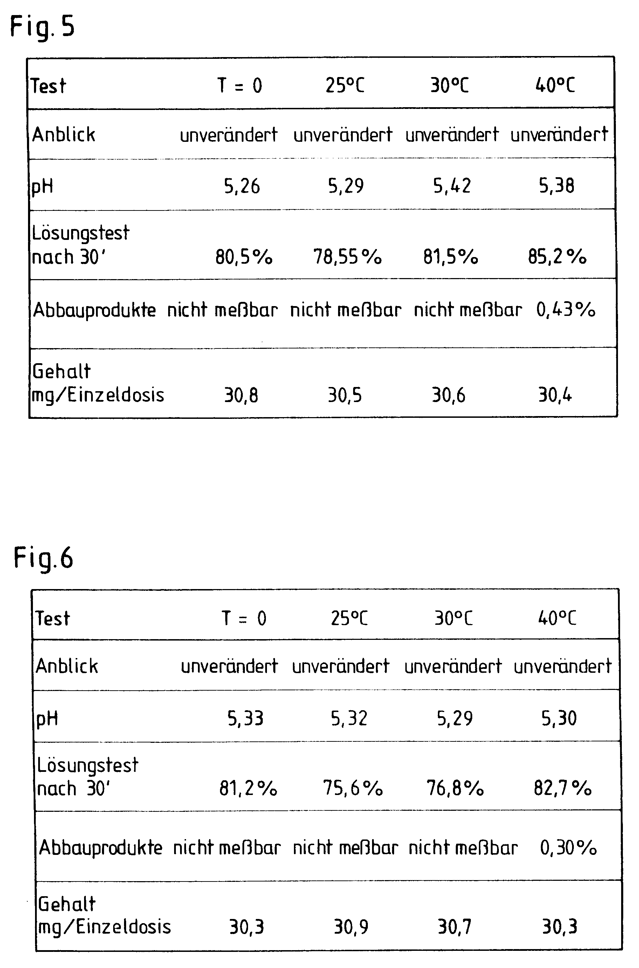

Die mit dem Nimodipin/PVP 25 Copräzipitat erhalten Ergebnisse sind in Figur 1 wiedergegeben. Sie beweisen, daß eine direkte Abhängigkeit der Wirkstoffmenge, die freigesetzt wird, von der PVP-Menge, die im Copräzipitat vorliegt, besteht. Bei Anwendung des Verhältnisses 1 : 3 wurde ein sehr zufriedenstellendes Auflösungsprofil erhalten, wobei eine 80%ige Auflösung nach 15 Minuten erreicht wurde, die sich für mehr als 2 Stunden praktisch stabil erwies.

Die obengenannten Werte deuten auf eine Übersättigung mit geringer, oder sogar keiner, Neigung zur Rekristallisierung. Höhere PVP-Mengen sind daher unter diesem Gesichtspunkt überflüssig, während sie zu einer unerwünschten Überflüssigen Schaumbildung führen könnten . Der vorliegenden Erfindung gemäß können auch andere PVP-Typen, wie z.B. PVP 30 und PVP 90, eingesetzt werden.

Im Vergleich zu PVP 25 zeigen die Ergebnisse in Figur 2, daß bei dem Verhältnis Nimodipin : PVP 30 = 1 : 2 höhere Konzentrationen erhalten werden, bei dem Verhältnis 1 : 3 dagegen kein Unterschied vorliegt.

Das mit PVP 90 erhaltene Copräzipitat stellt ein drittes Beispiel dar. In diesem Falle wurden nur Copräzipitate mit Verhältnis 1 : 1 bzw. 1 : 2 hergestellt, denn beim Verhältnis 1 : 3 wird eine nicht verwendbare plastische Masse erhalten.

Mit PVP 90 wird beim Verhältnis 1 : 1 ein gutes Profil erhalten, das beim Verhältnis 1 : 2 unveränderbar zu sein scheint.

Das bevorzugte Polyvinylpyrrolidon ist nach der vorliegenden Erfindung PVP 25; das bevorzugte Nimodipin : PVP Verhältnis ist 1 : 3.

Was das Orangensaft-Granulat betrifft, kann es mit verschiedener Konzentration mittels eines Fließbett-Granulators hergestellt werden, indem man auf ganz feiner Saccharose Orangensaft-Konzentrat sprüht. Das Brausestoffpaar wurde so eingestellt, daß das fertige Getränk einen pH-Wert zwischen 5 und 6 aufwies, der ein optimales Bereich zur Erhaltung eines angenehmen Geschmacks bei solchen Zubereitungen darstellt.

Nachfolgend wird die Erfindung durch einige beispielhafte Zubereitungen auf Basis von pharmazeutisch wirksamen Dihydropyridinen in Brausegranulatform erläutert. In den Beispielen sind die Teile als Gewichtsteile und die Verhältnisse als Gewicht/Gewicht-Angaben angeführt.

Beispiel 1

| | |
|---|---|
| Copräzipitat aus Nimodipin : PVP 25 (1:3) | 120,0 mg |
| Zitronensäure | 800,0 mg |
| Na-Bicarbonat | 800,0 mg |
| Zitrusaroma | 50,0 mg |
| Orangengranulat* q.s. ad | 3,5 g |
| * Beispielhafte Zusammensetzung des Orangengranulats: | |
| Saccharose | 1500,0 mg |
| Orangensaft (trocken) | 200,0 mg |
| Na-Saccharin | 7,0 mg |
| E 110 | 1,0 mg |

Nimodipin und PVP 25 werden im Verhältnis 1 : 3 in Aceton (4 Teile) bei Raumtemperatur gelöst; man rührt zur vollständigen Auflösung; nach Abdampfen des Acetons erhält man das Copräzipitat in der Form eines amorphen spröden Feststoffes, der anschließend granuliert und mit den anderen Bestandteilen vermischt wird.

Beispiel 2

| | |
|---|---|
| Copräzipitat aus Nimodipin : PVP 30 (1:2) | 90,0 mg |
| Zitronensäure | 800,0 mg |
| Na-Bicarbonat | 800,0 mg |
| Zitrusaroma | 50,0 mg |
| Orangengranulat* (s. oben) q.s. ad | 4,0 g |
| Man verfährt gemäß Beispiel 1. | |

Beispiel 3

| Copräzipitat aus Nifedipin : PVP 25 (1:3) | 40,0 mg |
|---|---|
| Zitronensäure | 800,0 mg |
| Na-Bicarbonat | 800,0 mg |
| Zitrusaroma | 50,0 mg |
| Orangengranulat* (s. oben) q.s. ad | 4,0 g |
| Man verfährt gemäß Beispiel 1. | |

Beispiel 4

| Copräzipitat aus Nisoldipin: PVP 25 (1:3) | 40,0 mg |
|---|---|
| Zitronensäure | 800,0 mg |
| Na-Bicarbonat | 800,0 mg |
| Zitrusaroma q.s. ad | 3,5 g |
| Man verfährt gemäß Beispiel 1. | |

In den Figuren 5 und 6 sind die Ergebnisse, die die Stabilität der obengenannten Zubereitungen betreffen, wiedergegeben.

Beispiel 5

| Copräzipitat aus Nimodipin : PVP 25 (1:3) | 120,0 mg |
|---|---|
| Zitrusaroma | 50,0 mg |
| Zitronengranulat* (wie oben) q.s. ad | 5,0 g |
| Man verfährt gemäß Beispiel 1. | |

Beispiel 6

Herstellung eines Copräzipitates mittels eines Fließbett-Granulators unter vermindertem Druck.

Nimodipin (1 Teil) und PVP 25 (3 Teile) werden in Aceton (8 Teile) bei Raumtemperatur gelöst; man rührt zur vollständigen Lösung; anschließend wird die Lösung in einem Fließbett-Apparat unter vermindertem Druck mit Rückgewinnung des Lösungsmittels unter folgenden Bedingungen sprühgetrocknet:

| Eintrittstemperatur | 120°-150°C |
|---|---|
| Ausgangstemperatur | 50°-80°C |
| Sprühdurchsatz | 1-1,5 kg/min |

Beispiel 7

Herstellung eines Copräzipitates durch Gefriertrocknung.

Nimodipin (5 Teile) und PVP 25 (15 Teile) werden in tert.-Butylalkohol (80 Teile) gelöst. Danach wird der Ansatz bei -10°C bis -20°C gefroren und gemäß üblichen Verfahren lyophilisiert.

Primärtrocknung: allmähliche Erwärmung bis + 20°C.

Sekundärtrocknung: 4-5 Std bei + 40°C

Beispiel 8

| Copräzipitat aus Nimodipin : Poloxamer F 127 (1:3) | 120,0 mg |
|---|---|
| E 110 | 1,0 mg |
| Saccharin | 7,0 mg |
| Zitrusaroma | 50,0 mg |
| Saccharose   q.s.ad | 3,5 g |

Das Nimodipin / Poloxamer F 127 (1:3) Copräzipitat wird in Aceton (5 Teile) gelöst; die dabei erhaltene Lösung wird zur Granulierung von feinster Saccharose (22,5 Teile) eingesetzt. Die Mischung wird gesiebt und unter vermindertem Druck getrocknet.

Der Rückstand wird granuliert und mit weiterer Saccharose und Zitrusaroma vermischt.

**Patentansprüche**

1. Als Getränk verabreichbare pharmazeutische Zubereitung auf der Basis von therapeutisch wirksamem Dihydropyridin, dadurch gekennzeichnet, daß sie aus einem Copräzipitat von wesentlich amorphem Dihydropyridin mit einem geeigneten pharmakologisch unbedenklichen Polymerisat besteht.

2. Pharmazeutische Zubereitung gemäß Anspruch 1, dadurch gekennzeichnet, daß das therapeutisch wirksame Dihydropyridin Nimodipin ist.

3. Pharmazeutische Zubereitung gemäß Anspruch 1 oder 2, dadurch gekennzeichnet, daß das pharmakologisch unbedenkliche Polymerisat Polyvinylpyrrolidon ist.

4. Pharmazeutische Zubereitung gemäß einem der Ansprüche 1 bis 3, dadurch gekennzeichnet, daß das Polyvinylpyrrolidon ein Molekulargewicht zwischen 25.000 und 90.000 Dalton aufweist.

5. Pharmazeutische Zubereitung gemäß einem der Ansprüche 1 bis 4, dadurch gekennzeichnet, daß das Gewichtsverhältnis Dihydropyridin / Polyvinylpyrrolidon zwischen 1:2 und 1:5 liegt.

6. Pharmazeutische Zubereitung gemäß Anspruch 1, dadurch gekennzeichnet, daß sie der folgenden Zusammensetzung entspricht:

| Copräzipitat aus Nimodipin : PVP 25 (1:3) | 120,0 mg |
|---|---|
| Zitronensäure | 800,0 mg |
| Na-Bicarbonat | 800,0 mg |
| Zitrusaroma | 50,0 mg |
| Orangengranulat   q.s.ad | 3,5 g |

7. Verfahren zur Herstellung einer pharmazeutischen Zubereitung in Getränkform, dadurch gekennzeichnet, daß man ein Copräzipitat aus Dihydropyridin und Polyvinylpyrrolidon herstellt, wobei man das Dihydropyridin und das Polyvinylpyrrolidon in einem geeigneten Lösungsmittel löst, anschließend das Copräzipitat nach an sich bekannten Verfahren so trocknet, daß das Dihydropyridin in einer wesentlich amorphen Form erhalten, und das so erhaltene Copräzipitat nach an sich bekannten Methoden in die gewünschte pharmazeutische Form überführt wird.

# Fig.1

% Auflösung

Nimo:PVP 25    1:3

Nimo:PVP 25    1:2

Nimo:PVP 25    1:1

Zeit (Min)

# Fig.2

% Auflösung

Nimo:PVP 30    1:3

Nimo:PVP 30    1:2

Nimo:PVP 30    1:1

Zeit (Min)

# Fig.3

% Auflösung

Nimo:PVP 90    1:2

Nimo:PVP 90    1:1

Zeit (Min)

# Fig.4

% Auflösung

Getestete Formulierungen
•—Tropfen +—Brausegranulat *—Tabletten

Zeit (Min)

## Fig. 5

| Test | T = 0 | 25°C | 30°C | 40°C |
|---|---|---|---|---|
| Anblick | unverändert | unverändert | unverändert | unverändert |
| pH | 5,26 | 5,29 | 5,42 | 5,38 |
| Lösungstest nach 30' | 80,5% | 78,55% | 81,5% | 85,2% |
| Abbauprodukte | nicht meßbar | nicht meßbar | nicht meßbar | 0,43% |
| Gehalt mg/Einzeldosis | 30,8 | 30,5 | 30,6 | 30,4 |

## Fig. 6

| Test | T = 0 | 25°C | 30°C | 40°C |
|---|---|---|---|---|
| Anblick | unverändert | unverändert | unverändert | unverändert |
| pH | 5,33 | 5,32 | 5,29 | 5,30 |
| Lösungstest nach 30' | 81,2% | 75,6% | 76,8% | 82,7% |
| Abbauprodukte | nicht meßbar | nicht meßbar | nicht meßbar | 0,30% |
| Gehalt mg/Einzeldosis | 30,3 | 30,9 | 30,7 | 30,3 |